(19) Europäisches Patentamt
European Patent Office
Office européen des brevets

(11) EP 1 760 074 A1

(12) **EUROPEAN PATENT APPLICATION**

(43) Date of publication:
**07.03.2007 Bulletin 2007/10**

(21) Application number: **06017885.2**

(22) Date of filing: **28.08.2006**

(51) Int Cl.:
***C07C 319/20*** *(2006.01)* ***C07C 319/28*** *(2006.01)*
***C07C 323/58*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **29.08.2005 JP 2005247169**

(71) Applicant: **Sumitomo Chemical Company, Limited**
**Tokyo 104-8260 (JP)**

(72) Inventors:
- **Shiozaki, Tetsuya**
  **Saijo-shi**
  **Ehime-ken (JP)**
- **Inoue, Go**
  **Niihama-shi**
  **Ehime-ken (JP)**

(74) Representative: **Vossius & Partner**
**Siebertstrasse 4**
**81675 München (DE)**

(54) **Process for producing methionine**

(57) A process for producing methionine by (1) hydrolyzing 5-[2-(methylthio)ethyl]-2,9-imidazolinedione in the presence of a basic potassium compound; (2) precipitating methionine by introducing $CO_2$ into the reaction solution from the step (1) and separating the slurry into the precipitate and a mother liquid; (3) mixing the mother liquid from the step (2) with an alcohol, precipitating methionine and $KHCO_3$ by introducing $CO_2$ into the mixture, and separating the slurry into the precipitate and a mother liquid; and (4) concentrating the mother liquid from the step (3), precipitating methionine and $KHCO_3$ by introducing $CO_2$ into the concentrated solution, and separating the slurry into the precipitate and a mother liquid.

EP 1 760 074 A1

## Description

The present invention relates to a process for producing methionine by hydrolyzing 5-[(2-(methylthio) ethyl)]-2,9-imidazolinedione according to the following reaction formula (1):

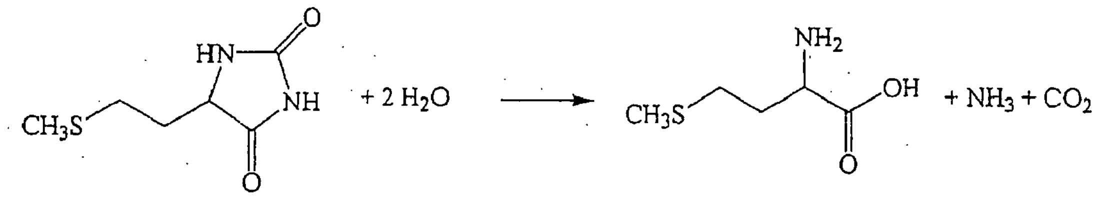

(1)

Methionine is useful as an additive for animal feeding stuffs.

One of the conventional processes for producing methionine comprises hydrolyzing 5-[(2-(methylthio)ethyl)]-2,4-imidazolinedione under basic conditions using a basic potassium compound such as potassium carbonate and potassium hydrogen carbonate. In this process, methionine can be separated and obtained in the form of a crystal through crystallization by introducing carbon dioxide into a reaction solution after hydrolysis. However, a mother liquid after the separation of methionine still contains methionine in an amount corresponding to its solubility in a reaction medium and also potassium hydrogen carbonate which may be recycled as the basic potassium compound. Therefore, it is preferable to recycle the mother liquid to the hydrolysis step. In such a case, if the entire amount of the mother liquid is recycled, impurities are accumulated in the reaction mixture of the hydrolysis step, and thus it is required to purge the mother liquid at a prescribed rate. In addition, the disposal of the purged mother liquid as a waste liquid is not a good measure since it results in the loss of methionine and potassium hydrogen carbonate contained therein and the load and cost of disposal of the waste liquid are high.

Accordingly, various processes for recovering the second crystal of methionine and potassium hydrogen carbonate from the mother liquid have been proposed. For example, JP-B-54-9174 discloses crystallization by mixing a water-soluble solvent such as an alcohol (e.g. methanol, etc.) or acetone with the mother liquid and introducing carbon dioxide into the mixture. JP-A-51-1415 discloses crystallization by concentrating the mother liquid and introducing carbon dioxide into the concentrated liquid. Further, JP-A-5-320124 discloses crystallization by mixing the mother liquid with isopropanol and introducing carbon dioxide into the mixture.

In the conventional processes described above, the mother liquid after the separation of the second crystal is disposed as a waste liquid. However, the mother liquid still contains methionine and potassium hydrogen carbonate. Thus, if they can be recovered in the form of a third crystal, it is advantageous from the viewpoint of the production cost, and the load and cost of disposal of the waste liquid can be lowered.

Accordingly, an object of the present invention is to provide a process for producing methionine, which is advantageous from the viewpoint of the cost and also of the disposal of waste liquid, by efficiently recovering even the third crystal.

It was found that the above object can be achieved by obtaining methionine by carrying out crystallization after the hydrolysis of 5-[(2-(methylthio)ethyl)]-2,4-imidazolinedione and subsequently recovering second and third crystals from the mother liquid after the separation of methionine in the prescribed manner.

That is, the present invention provides a process for producing methionine comprising the following steps (1) to (4) :

(1) hydrolyzing 5-[2-(methylthio)ethyl]-2,4-imidazolinedione in the presence of a basic potassium compound (Reaction step (1));

(2) precipitating methionine by introducing carbon dioxide into the reaction solution obtained in the step (1) and separating the resulting slurry into the precipitate and a mother liquid (First crystallization step (2));

(3) mixing the mother liquid obtained in the step (2) with a lower alcohol, precipitating methionine and potassium hydrogen carbonate by introducing carbon dioxide into the mixed solution, and separating the resulting slurry into the precipitate and a mother liquid (Second crystallization step (3)); and

(4) concentrating the mother liquid obtained in the step (3), precipitating methionine and potassium hydrogen carbonate by introducing carbon dioxide into the concentrated solution, and separating the resulting slurry into the precipitate and a mother liquid (Third crystallization step (4)).

According to the process of the present invention, methionine can be produced advantageously from the viewpoint of the cost and also of the disposal of waste liquid treatment.

In the present invention, 5-[(2-(methylthio)ethyl)]-2,4-imidazolinedione is used as a starting material and it is

hydrolyzed in the presence of a basic potassium compound to obtain a reaction solution containing methionine in the form of a potassium salt [the reaction step (1)].

The starting material, 5-[(2-(methylthio)ethyl)]-2,4-imidazolinedione, can be prepared by, for example, the reaction of 2-hydroxy-4-methylthiobutanenitrile with ammonia and carbon dioxide, or ammonium carbonate according to the reaction formula (2) or (3):

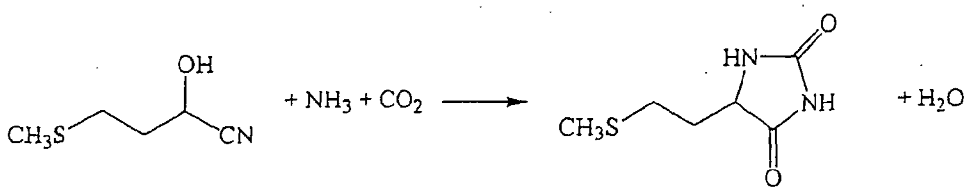

(2)

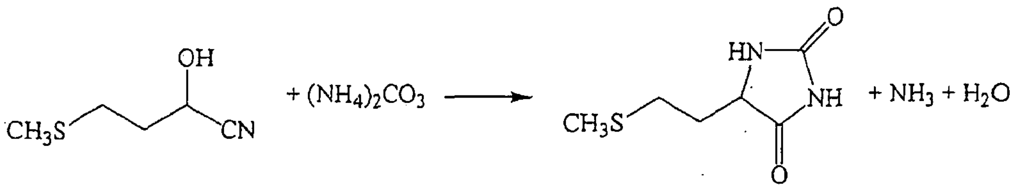

(3)

Examples of the basic potassium compound include potassium hydroxide, potassium carbonate, and potassium hydrogen carbonate. They may be used as a mixture of two or more of them, if desired. The amount of the basic potassium compound is, in term of potassium, usually 2 to 10 moles, preferably 3 to 6 moles, per mole of 5-[(2-(methylthio)ethyl)]-2,4-imidazolinedione.

The weight of water used is usually 2 to 20 times the weight of 5-[(2-(methylthio)ethyl)]-2,4-imidazolinedione.

The hydrolysis reaction may be carried out under heating at about 150 to 200°C under pressure at about 0.5 to 1 MPa (gauge pressure). The reaction time is usually in a range from 10 minutes to 24 hours.

To recover methionine obtained in the hydrolysis step from the hydrolysis reaction solution, crystallization is carried out by introducing carbon dioxide into the reaction solution and the precipitated methionine is recovered as a first crystal by carrying out solid-liquid separation of the resulting slurry by filtration, decantation or the like [the first crystallization step (2)].

The first crystallization may be carried out under pressure usually at about 0.1 to 1 MPa (gauge pressure), preferably about 0.2 to 0.5 MPa (gauge pressure) of carbon dioxide gas by allowing carbon dioxide gas to be absorbed in the hydrolysis reaction solution. The crystallization temperature is usually 0 to 50°C, preferably 10 to 30°C. The crystallization time may be adjusted based on a duration in which the carbon dioxide gas is saturated in the hydrolysis reaction solution and methionine is sufficiently precipitated, and it is usually in a range from 30 minutes to 24 hours.

Methionine obtained by the solid-liquid separation is dried after being washed or subjected to pH adjustment, if necessary, to obtain a product. The drying may be carried out by heating at about 50 to 120°C in slightly reduced pressure and the drying time may be usually in a range from 10 minutes to 24 hours.

In the mother liquid after the methionine separation (hereinafter, this mother liquid is sometimes referred to as "first crystal mother liquid"), methionine may remain in an amount corresponding to its solubility in the reaction medium, and also potassium hydrogen carbonate possible to be recycled is contained as the basic potassium compound. Therefore, the first crystal mother liquid is preferably recycled to the hydrolysis reaction in the step (1) However, the first crystal mother liquid may contain impurities contained in the starting material or produced due to side reactions during the hydrolysis, for example, amino acids other than methionine (e.g. glycine and alanine) and coloring components. These impurities are brought into the hydrolysis reaction by recycling. Therefore, it is necessary to recycle the first crystal mother liquid not entirely but partially to an extent that the impurities are not accumulated. Thus, a proportion of the first crystal mother liquid to be recycled is usually 50 to 90% by weight, preferably 70 to 90% by weight of the entire amount of the first crystal mother liquid.

In a preferable embodiment, the first crystal mother liquid is concentrated and then the concentrated liquid is recycled. As the result of the concentration, carbon dioxide can be removed from the first crystal mother liquid and thus the recycling liquid, which has an increased basicity and is advantageous for the hydrolysis reaction, can be obtained.

When the concentration is carried out at a high temperature of about 100 to 150°C,the conversion reaction of potassium hydrogen carbonate in the first crystal mother liquid into potassium carbonate ($2KHCO_3 \rightarrow K_2CO_3 + H_2O +$

$CO_2$) is promoted and the recycling solution, which is more advantageous for the hydrolysis because of the further increased basicity, can be obtained. The concentration can be carried out under atmospheric pressure, a reduced pressure or an elevated pressure. To carry out the concentration at the high temperature described above, it is preferable to use an elevated pressure. The concentration ratio is usually 1.2 to 4 times, preferably 1.5 to 3.5 times. Herein, a concentration ratio means the ratio of the weight of a solution before concentration to the weight of the solution after concentration [(solution weight before concentration)/(solution weight after concentration)].

The unrecycled portion of the first crystal mother liquid is subjected to crystallization to recover methionine and potassium hydrogen carbonate as a second crystal. In the present invention, this crystallization step is carried out to recover precipitated methionine and potassium hydrogen carbonate as a second crystal by mixing the first crystal mother liquid with a lower alcohol, introducing carbon dioxide into the mixture, and carrying out the solid-liquid separation of the resulting slurry by filtration, decantation or the like [second crystallization step (3)]. Alternatively, the first crystal mother liquid as a whole may be subjected to crystallization without recycling a part of the first crystal mother liquid.

Similarly to the first crystallization, this second crystallization is preferably carried out by allowing carbon dioxide gas to be absorbed in the mixture of the first crystal mother liquid and the lower alcohol under pressure usually at about 0.1 to 1 MPa (gauge pressure), preferably about 0.2 to 0.5 MPa (gauge pressure) of carbon dioxide gas. The crystallization temperature is usually 0 to 50°C, preferably 5 to 20°C. The crystallization time may be adjusted based on a duration in which the carbon dioxide gas is saturated in the above mixture, and methionine and potassium hydrogen carbonate are sufficiently precipitated, and it is usually in a range from 10 minutes to 24 hours.

In general, an alkanol having 1 to 5 carbon atoms is used as a lower alcohol. In particular, a lower alcohol which can be mixed with water at any ratio is preferably used, and examples of such a lower alcohol include methanol, ethanol, n-propanol, isopropanol, and tert-butanol. Among them, isopropanol is particularly preferable. The weight of the lower alcohol used is usually 0.05 to 5 times, preferably 0.1 to 2 times the weight of the first crystal mother liquid to be subjected to the crystallization. In this case, the first crystal mother liquid and the lower alcohol may be mixed before or during introducing carbon dioxide gas.

The first crystal mother liquid to be subjected to the second crystallization is preferably the concentrated first crystal mother liquid, as in the case of the first crystal mother liquid to be recycled. The recovery rate of the second crystal can be increased by concentration. The concentration can be carried out under the same conditions as those employed in the concentration of the first crystal mother liquid to be recycled. Alternatively, the whole amount of the first crystal mother liquid is concentrated and then the concentrated mother liquid is separated into one portion of the mother liquid for recycling and the other for second crystallization. In the case where the first crystal mother liquid is concentrated and then subjected to the second crystallization, the weight of the lower alcohol is usually 0.05 to 5 times, preferably 0.1 to 2 times the weight of the concentrated mother liquid.

Since the basicity of the mother liquid is increased by concentration, when the concentrated mother liquid is heated, the regeneration of methionine is preferably promoted by the hydrolysis of methionine dipeptide (i.e. a dehydration condensate of two methionine molecules) contained in the liquid. The heating is preferably carried out at a temperature of about 140 to 180°C under pressure at about 0. 5 to 2 MPa (gauge pressure). The heating time is usually 10 minutes to 24 hours.

The recovered second crystal may be recycled to the hydrolysis reaction of the step (1). In such a case, it is preferable to dissolve the second crystal in the first crystal mother liquid for recycling, from the viewpoint of handling.

The mother liquid after the second crystal separation (hereinafter, this mother liquid is sometimes referred to as "second crystal mother liquid") still contains methionine and potassium hydrogen carbonate. Therefore, in the present invention, for the purpose of additional recovery of methionine and potassium hydrogen carbonate from the second crystal mother liquid as a third crystal, the second crystal mother liquid is concentrated, and carbon dioxide is introduced into the concentrated mother liquid for crystallization. Then, the resulting slurry is subjected to solid-liquid separation by filtration, decantation or the like to recover precipitated methionine and potassium hydrogen carbonate as the third crystal [third crystallization step (4)].

The concentration of the second crystal mother liquid may be carried out under an atmospheric pressure, a reduced pressure, or an elevated pressure. Preferably, the concentration is carried out under a reduced pressure, since the temperature of a heat source can be lowered. The concentration ratio is usually 1.2 to 4 times, preferably 1.5 to 3.5 times. The concentration of the second crystal mother liquid makes it possible to remove the lower alcohol from the second crystal mother liquid and increase the recovery rate of the third crystal.

Next, the concentrated solution of the second crystal mother liquid is subjected to the crystallization. Similarly to the first crystallization and the second crystallization, the third crystallization is preferably carried out under pressure usually at about 0.1 to 1 MPa (gauge pressure), preferably about 0.2 to 0.5 MPa (gauge pressure) of carbon dioxide gas by allowing carbon dioxide to be absorbed in the concentrated mother liquid. The crystallization temperature is usually 0 to 50°C, preferably 5 to 30°C. The crystallization time may be adjusted based on a duration in which the carbon dioxide gas is saturated in the concentrated mother liquid and methionine and potassium hydrogen carbonate are sufficiently precipitated, and it is usually in a range from 10 minutes to 24 hours.

The third crystallization is preferably carried out in the presence of polyvinyl alcohol, as described in JP-A-4-169570. Such a treatment method can precipitate the third crystal in such a state that the crystal is easily separated from the mother liquid. Thus, the mother liquid hardly remains in the third crystal during the subsequent solid-liquid separation, and the impurity content in the recovered third crystal can be decreased. The amount of the polyvinyl alcohol is usually 100 to 5000 ppm by weight, preferably 200 to 3000 ppm by weight, based on the weight of the concentrated second crystal mother liquid.

The first crystallization and the second crystallization may also be carried out in the presence of polyvinyl alcohol. In particular, when the first crystallization is carried out in the presence of polyvinyl alcohol, preferably, methionine with good powder properties can be obtained.

The recovered third crystal may preferably be recycled to the hydrolysis reaction of the step (1), similarly to the case of the second crystal.

All of the above-mentioned steps (1) to (4) may be carried out continuously or in a batch manner. Alternatively, some of the steps may be carried out continuously and the rest may be carried out in a batch manner.

## Examples

Hereinafter, the invention will be illustrated by the following Examples, which do not limit the present invention in any way. In the Examples, % and parts expressing concentrations or amounts are based on weight unless otherwise specified.

### Example 1

Reaction step (1)

A hydrolysis reaction was carried out at a temperature of 173 to 178°C under a pressure of 0.88 MPa (gauge pressure) for a residence time of 1 hour by introducing, into a reaction vessel, 100 parts/hr of an aqueous solution containing 18.7% of 5-[(2-(methylthio)ethyl)]-2,4-imidazolinedione, 1.0 part/hr of potassium hydroxide, 67.6 parts/hr of the primarily concentrated liquid of the first crystal mother liquid described below, and 25.8 parts/hr of the solution of second crystal described below.

First crystallization step (2)

The reaction solution (133.1 parts/hr) obtained by the previous hydrolysis reaction was mixed with 60.7 parts/hr of water and 0.023 parts/hr of polyvinyl alcohol and introduced into a crystallization apparatus. Then, the crystallization was carried out at 20°C under a pressure of 0.3 MPa (gauge pressure) of carbon dioxide gas to precipitate methionine. The resulting slurry was filtered, and the filtration residue was washed with water and then dried at 85 to 105°C under a slightly reduced pressure to obtain 15.6 parts/hr of methionine (purity: 99.6%; yield: 97%). As a filtrate, 184.0 parts/hr of a first crystal mother liquid was recovered.

The first crystal mother liquid obtained in the above process (184.0 parts/hr) was introduced into a concentration apparatus and concentrated at 115°C and then 140°C under a pressure of 0.2 MPa (gauge pressure) to obtain 106.4 parts/hr of a primarily concentrated liquid (the primary concentration ratio: 1.7 times). The analysis of the primarily concentrated liquid revealed that the methionine concentration was 6.0% and the potassium concentration was 13.5%.

As described above, 67.6 parts/hr of the primarily concentrated liquid (106.4 parts/hr) of the above-mentioned first crystal mother liquid was recycled to the hydrolysis reaction. Also, 18.5 parts/hr of the solution was introduced into a heating apparatus and heated at 165°C under a pressure of 1 MPa (gauge pressure) for a residence time of 1 hour and then introduced into a concentration apparatus and concentrated at 135°C under a pressure of 0.2 MPa (gauge pressure) to obtain 12.3 parts/hr of a secondarily concentrated liquid (secondary concentration ratio: 1.5 times; cumulative ratio of the primary and secondary concentrations: 2.6 times). Further, 20.3 parts/hr of the remaining was used for dissolution of a wet cake of the second crystal as described below. Secondary crystallization step (3)

The secondarily concentrated liquid of the above-mentioned first crystal mother liquid (12. 3 parts/hr) was mixed with 3.3 parts/hr of isopropanol and introduced into the crystallization apparatus. Then, the crystallization was carried out at 12 to 16°C under a pressure of 0.3 MPa (gauge pressure) of carbon dioxide gas. The resulting slurry was filtered to obtain 7.8 parts/hr of a wet cake of the second crystal as a filtration residue. Also, as a filtrate, 9.1 parts/hr of the second crystal mother liquid was recovered.

The wet cake of the second crystal (7.8 parts/hr) from the previous step was dissolved in the remainder (20.3 parts/hr) of the above-mentioned primarily concentrated liquid of the first crystal mother liquid, and the solution was introduced into a concentration apparatus and concentrated at 80°C under atmospheric pressure to remove isopropanol contained in the second crystal to obtain 25.8 parts/ hour of a second crystal solution. The analysis of the second crystal

solution revealed that the methionine concentration was 7.6% and the potassium concentration was 18.2%. The second crystal solution (25.8 parts/hr) was recycled to the hydrolysis reaction as described above.

Tertiary crystallization step (4)

The second crystal mother liquid (9.1 parts/hr) from the previous step was introduced into a concentration apparatus and concentrated at 80 to 110°C under atmospheric pressure to distill isopropanol off to obtain 6.0 parts of a primarily concentrated liquid (primary concentration ratio: 1.5 times). The analysis of the primarily concentrated liquid revealed that the methionine concentration was 3.14%, the concentrations of two diastereomers of methionine dipeptides (hereinafter, referred to as MDP-1 and MDP-2, respectively) were 1.55% for MDP-1 and 1.68% for MDP-2, and the potassium concentration was 7.25%.

A portion of the primarily concentrated liquid of the second crystal mother liquid was taken and introduced into a concentration apparatus and concentrated at 60°C under a reduced pressure of absolute pressure of 60 mmHg (8 kPa) until the secondary concentration ratio reached 2.3 times (cumulative ratio of the primary and the secondary concentration: 3.5 times). The analysis of the secondarily concentrated liquid revealed that the glycine concentration was 0.69% and the alanine concentration was 1.07%.

The secondarily concentrated liquid of the second crystal mother liquid was introduced into a crystallization apparatus and crystallized at 10°C under a pressure of 0.3 MPa (gauge pressure) of carbon dioxide gas to precipitate methionine and potassium hydrogen carbonate. The resulting slurry was filtered, and the filtration residue was recovered as a wet cake of the third crystal. The respective concentrations of methionine, MDP-1, MPD-2, and potassium, which are valuable components, in the wet cake were analyzed, and the recovery rates of the respective valuable components were measured according to the following calculation formula to find that the recovery rate of methionine was 82.5%; that of MDP-1 was 51.4%; that of MDP-2 was 91.1%; and that of potassium was 49.4%.

Recovery rate of a valuable component (%) =

100 x [recovery amount of wet cake (part) × concentration of a valuable component in wet cake (%)]/[amount of primarily concentrated liquid of second crystal mother liquid (part) × concentration of a valuable component in primarily concentrated liquid of second crystal mother liquid (%)]

The respective concentrations of glycine and alanine, which are impurities in the wet cake, were analyzed and the uptake rate of each impurity was calculated according to the following calculation formula to find that the uptake rate of glycine was 35.6% and that of alanine was 33.6%.

Uptake rate of an impurity (%) =

100 x [recovery amount of wet cake (part)×concentration of an impurity in wet cake (%)]/[use amount of secondarily concentrated liquid of second crystal mother liquid (part)×concentration of an impurity in secondarily concentrated liquid of second crystal mother liquid (%)]

Examples 2 to 4

The same procedures as those of Example 1 were repeated, except that the ratio of the secondary concentration in the third crystallization step (4) of Example 1 was changed to the ratios shown in Table 1.

The respective recovery rates of the valuable components in the obtained wet cake of the third crystal were measured and shown in Table 1 together with the results of Example 1.

Table 1

| Example No. | Secondary Concentration Ratio (times) | Recovery Rate (%) | | | |
|---|---|---|---|---|---|
| | | Methionine | MDP-1 | MDP-2 | Potassium |
| 2 | 1.0 | 40.5 | 5.2 | 61.0 | 4.4 |
| 3 | 1.4 | 61.5 | 11.1 | 75.8 | 18.4 |
| 4 | 1.8 | 75.3 | 13.7 | 84.7 | 33.5 |
| 1 | 2.3 | 82.5 | 51.8 | 91.1 | 49.4 |

Examples 5 to 7

The same procedures as those of Example 1 were repeated , except that the temperature of the crystallization in the third crystallization step (4) of Example 1 was changed to the temperatures shown in Table 2. The respective recovery rates of the valuable components in the obtained wet cake of the third crystal were measured and shown in Table 2 together with the results of Example 1.

Table 2

| Example No. | Crystallization Temperature (°C) | Recovery Rate (%) | | | |
|---|---|---|---|---|---|
| | | Methionine | MDP-1 | MDP-2 | Potassium |
| 1 | 10 | 82.5 | 51.8 | 91.1 | 49.4 |
| 5 | 15 | 82.6 | 43.7 | 80.5 | 50.6 |
| 6 | 20 | 80.4 | 46.2 | 82.3 | 49.8 |
| 7 | 25 | 82.5 | 49.1 | 88.5 | 51.2 |

Examples 8 to 10

The same procedures as those of Example 1 were repeated, except that the gauge pressure of carbon dioxide gas in the third crystallization step (4) of Example 1 was changed to the pressures shown in Table 3. The respective recovery rates of the valuable components in the obtained wet cake of the third crystallization were measured and shown in Table 3 together with the results of Example 1.

Table 3

| Example No. | Gauge Pressure (MPa) | Recovery Rate (%) | | | |
|---|---|---|---|---|---|
| | | Methionine | MDP-1 | MDP-2 | Potassium |
| 8 | 0.2 | 83.5 | 43.7 | 80.5 | 47.3 |
| 1 | 0.3 | 82.5 | 51.8 | 91.1 | 49.4 |
| 9 | 0.4 | 81.3 | 46.3 | 86.2 | 47.2 |
| 10 | 0.5 | 75.0 | 41.4 | 80.4 | 44.9 |

Examples 11 to 15

The same procedures as those of Example 1 were repeated, except that the crystallization was carried out by adding polyvinyl alcohol in an amount shown in Table 4 to the secondary concentrated solution of the second crystal mother liquid in the third crystallization step (4) of Example 1. The respective recovery rates of the valuable components and the uptake rates of the respective impurities in the obtained wet cake of the third crystal were measured and shown in Table 4 together with the results of Example 1.

Table 4

| Example No. | Polyvinyl Alcohol (wt. ppm) | Recovery Rate (%) | | | | Uptake Rate (%) | |
|---|---|---|---|---|---|---|---|
| | | Methionine | MDP-1 | MDP-2 | Potassium | Glycine | Alanine |
| 1 | - | 82.5 | 51.8 | 91.1 | 49.4 | 35.6 | 33.6 |
| 11 | 250 | 83.8 | 13.4 | 90.1 | 36.0 | 17.1 | 15.3 |
| 12 | 500 | 80.2 | 12.8 | 84.1 | 38.9 | 14.8 | 15.2 |
| 13 | 1000 | 78.8 | 13.9 | 81.6 | 42.6 | 10.9 | 12.8 |
| 14 | 2000 | 85.3 | 14.7 | 95.6 | 35.1 | 16.4 | 16.2 |
| 15 | 3000 | 74.7 | 15.0 | 83.3 | 37.7 | 16.0 | 15.0 |

## Claims

**1.** A process for producing methionine comprising the following steps (1) to (4):

(1) hydrolyzing 5-[2-(methylthio)ethyl]-2,4-imidazolinedione in the presence of a basic potassium compound (Reaction step (1));
(2) precipitating methionine by introducing carbon dioxide into the reaction solution obtained in the step (1) and separating the resulting slurry into the precipitate and a mother liquid (First crystallization step (2));
(3) mixing the mother liquid obtained in the step (2) with a lower alcohol, precipitating methionine and potassium hydrogen carbonate by introducing carbon dioxide into the mixture, and separating the resulting slurry into the precipitate and a mother liquid (Second crystallization step (3)); and
(4) concentrating the mother liquid obtained in the step (3), precipitating methionine and potassium hydrogen carbonate by introducing carbon dioxide into the concentrated solution, and separating the resulting slurry into the precipitate and a mother liquid (Third crystallization step (4)).

**2.** The process according to claim 1, wherein the mother liquid obtained in the step (2) is subjected to the step (3) after the mother liquid has been concentrated.

**3.** The process according to claim 1 or 2, wherein the lower alcohol used in the step (3) is selected from the group consisting of methanol, ethanol, n-propanol, isopropanol and tert-butanol.

**4.** The process according to any one of claims 1 to 3, wherein methionine and potassium hydrogen carbonate are precipitated in the presence of polyvinyl alcohol in the step (4).

**5.** The process according to any one of claims 1 to 4, wherein a portion of the mother liquid obtained in the step (2) is recycled to the step (1).

**6.** The process according to claim 5, wherein the mother liquid obtained in the step (2) is recycled to the step (1) after the mother liquid has been concentrated.

**7.** The process according to any one of claims 1 to 6, wherein the precipitate obtained in the step (3) is recycled to the step (1).

**8.** The process according to any one of claims 1 to 7, wherein the precipitate obtained in the step (4) is recycled to the step (1).

European Patent Office

# EUROPEAN SEARCH REPORT

Application Number
EP 06 01 7885

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 839 804 A2 (SUMITOMO CHEMICAL CO [JP]) 6 May 1998 (1998-05-06) | | INV. C07C319/20 C07C319/28 C07C323/58 |
| Y | * the whole document * claim 1 (steps A, B, D) | 1-8 | |
| Y | * page 3, line 47 * | 3 | |
| Y | claim 1 (step E) | 7,8 | |
| Y | claim 1 (step C) | 5 | |
| A,D | GB 1 296 347 A (DEGUSSA [DE]) 15 November 1972 (1972-11-15) | | |
| Y | * the whole document * * example 1 * * claims 1,2,5 * * page 2, column 2, line 112 * | 1-8 | |
| Y | * page 2, column 2, line 112 * | 3 | |
| Y | * page 2, column 2, lines 90-103 * | 5 | |
| A,D | US 4 303 621 A (LUSSLING THEODOR ET AL) 1 December 1981 (1981-12-01) | 1-8 | |
| Y | * column 3, lines 3-8 * | 1,2 | |
| Y | * column 1, lines 47-49 * | 6 | TECHNICAL FIELDS SEARCHED (IPC) |
| Y | * column 2, lines 26-32 * | 1,2 | C07C |
| A,D | JP 05 320124 A (SUMITOMO CHEMICAL CO) 3 December 1993 (1993-12-03) | | |
| Y | * paragraph [0007] * | 3 | |
| A | EP 1 457 486 A1 (NIPPON SODA CO [JP]) 15 September 2004 (2004-09-15) * page 4, paragraphs 15,16 * | 4 | |
| A,D | JP 02 921097 B2 (SUMITOMO CHEMICAL CO) 19 July 1999 (1999-07-19) * abstract * | 4 | |

The present search report has been drawn up for all claims

| Place of search | Date of completion of the search | Examiner |
|---|---|---|
| Munich | 3 January 2007 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

8
EPO FORM 1503 03.82 (P04C01)

**ANNEX TO THE EUROPEAN SEARCH REPORT ON EUROPEAN PATENT APPLICATION NO.** EP 06 01 7885

This annex lists the patent family members relating to the patent documents cited in the above-mentioned European search report.
The members are as contained in the European Patent Office EDP file on
The European Patent Office is in no way liable for these particulars which are merely given for the purpose of information.

03-01-2007

| Patent document cited in search report | Publication date | Patent family member(s) | Publication date |
|---|---|---|---|
| EP 0839804 A2 | 06-05-1998 | BR 9705174 A | 20-07-1999 |
| | | CN 1181378 A | 13-05-1998 |
| | | DE 69709526 D1 | 14-02-2002 |
| | | DE 69709526 T2 | 01-08-2002 |
| | | ES 2166036 T3 | 01-04-2002 |
| | | RU 2208943 C2 | 27-07-2003 |
| | | US 5945563 A | 31-08-1999 |
| GB 1296347 A | 15-11-1972 | AT 295489 B | 10-01-1972 |
| | | BE 745432 A1 | 16-07-1970 |
| | | CH 529118 A | 15-10-1972 |
| | | DE 1906405 A1 | 03-12-1970 |
| | | FR 2032788 A5 | 27-11-1970 |
| | | HU 162233 B | 29-01-1973 |
| | | IL 33840 A | 29-08-1973 |
| | | IT 954084 B | 30-08-1973 |
| | | JP 54009174 B | 21-04-1979 |
| | | NL 7001725 A | 11-08-1970 |
| | | RO 56492 A1 | 15-06-1975 |
| | | SE 373842 B | 17-02-1975 |
| | | SU 503509 A3 | 15-02-1976 |
| US 4303621 A | 01-12-1981 | AT 339271 B | 10-10-1977 |
| | | AT 333175 A | 15-02-1977 |
| | | BE 828648 A1 | 30-10-1975 |
| | | BR 7502660 A | 16-03-1976 |
| | | CA 1049555 A1 | 27-02-1979 |
| | | CH 611277 A5 | 31-05-1979 |
| | | DD 119226 A5 | 12-04-1976 |
| | | DE 2421167 A1 | 20-11-1975 |
| | | DK 190475 A | 03-11-1975 |
| | | ES 437156 A1 | 16-01-1977 |
| | | FR 2269522 A1 | 28-11-1975 |
| | | GB 1510024 A | 10-05-1978 |
| | | IL 47215 A | 31-05-1979 |
| | | JP 1051184 C | 26-06-1981 |
| | | JP 51001415 A | 08-01-1976 |
| | | JP 55042985 B | 04-11-1980 |
| | | NL 7505228 A | 04-11-1975 |
| | | SU 586836 A3 | 30-12-1977 |
| JP 5320124 A | 03-12-1993 | JP 3206103 B2 | 04-09-2001 |
| EP 1457486 A1 | 15-09-2004 | AU 2002354109 A1 | 10-06-2003 |
| | | CN 1589259 A | 02-03-2005 |
| | | WO 03045904 A1 | 05-06-2003 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 06 01 7885

This annex lists the patent family members relating to the patent documents cited in the above-mentioned European search report.
The members are as contained in the European Patent Office EDP file on
The European Patent Office is in no way liable for these particulars which are merely given for the purpose of information.

03-01-2007

| Patent document cited in search report | Publication date | Patent family member(s) | Publication date |
|---|---|---|---|
| EP 1457486 A1 | | US 2004267049 A1 | 30-12-2004 |
| JP 2921097 B2 | 19-07-1999 | JP 4169570 A | 17-06-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 54009174 B
- JP 51001415 A
- JP 5320124 A
- JP 4169570 A